# EUROPEAN PATENT APPLICATION

(11) **EP 1 902 678 A2**
(43) Date of publication of application: **26.03.2008**
(21) Application number: 07019513.6
(22) Date of filing: 01.07.2003
(51) Int. Cl.: A61B 17/12

(54) **Coaxial stretch-resistant vaso-occlusive device**

(30) Priority: 02.07.2002 US 188934
(62) Divisional of application: 03763052.2
(71) Applicant: Microvention, Inc., Aliso Viejo, CA 92656-1498 (US)
(72) Inventor: Schaefer, Dean, Laguna Hills, CA 92653 (US); Almazan, Horacio, Rancho Santa Margarita, CA 92688 (US)
(74) Representative: Tonnesen, Bo

(57) **Abstract**

A filamentous vaso-occlusive implant device includes an elongate, flexible outer member, preferably a microcoil, arranged coaxially around at least one inner member. When the outer member is subjected to axial tension, it elongates axially, while it simultaneously contracts radially. The radial contraction is resisted by the inner member, which thereby limits the elongation of the outer member so that its elastic limit is not exceeded. Therefore, permanent streching or deformation of the outer member is not permitted. An obturator tip is advantageously provided at the distal end of the device, and a coupling element for detachable attachment of the device to a delivery mechanism is advantageously attached to the proximal end of the device. The inner member may be a microcoil, a hollow tube, a solid filament, a spiral cut tube, a slotted tube, or a tubular braid, and it may be provided with a bioactive agent.

## Description

### CROSS-REFERENCE TO RELATED APPLICATION

This application is related to co-pending Application Serial No. 10/ ; filed July, 2002.

### FEDERALLY SPONSORED RESEARCH OR DEVELOPMENT

Not Applicable

### BACKGROUND OF THE INVENTION

The invention is generally related to the field of vascular occlusion and, more specifically, to vaso-occlusive devices that are resistant to stretching and kinking while maintaining high flexibility.

Vaso-occlusive devices are typically used within the vasculature of the human body to block the flow of blood through a vessel through the formation of an embolus. Vaso-occlusive devices are also used to form an embolus within an aneurysm stemming from the vessel. Vaso-occlusive devices can be formed of one or more elements, generally delivered into the vasculature via a catheter or similar mechanism.

The embolization of blood vessels is desired in a number of clinical situations. For example, vascular embolization has been used to control vascular bleeding, to occlude the blood supply to tumors, and to occlude vascular aneurysms, particularly intracranial aneurysms. In recent years, vascular embolization for the treatment of aneurysms has received much attention. Several different treatment modalities have been employed in the prior art.

One approach that has shown promise is the use of thrombogenic microcoils. These microcoils may be made of a biocompatible metal alloy (typically platinum and tungsten) or a suitable polymer. If made of metal, the coil may be provided with Dacron fibers to increase thrombogenicity. The coil is deployed through a microcatheter to the vascular site. Examples of microcoils are disclosed in the following U.S. patents: 4,994,069 - Ritchart et al.; 5,133,731 - Butler et al.; 5,226,911 - Chee et al.; 5,312,415 - Palermo; 5,382,259 - Phelps et al.; 5,382,260 - Dormandy, Jr. et al.; 5,476,472 - Dormandy, Jr. et al.; 5,578,074 - Mirigian; 5,582,619 - Ken; 5,624,461 - Mariant; 5,645,558 - Horton; 5,658,308 - Snyder; and 5,718,711 - Berenstein et al.

A specific type of microcoil that has achieved a measure of success is the Guglielmi Detachable Coil ("GDC"), described in U. S. Patent No. 5,122,136 - Guglielmi et al. The GDC employs a platinum wire coil fixed to a stainless steel delivery wire by a solder connection. After the coil is placed inside an aneurysm, an electrical current is applied to the delivery wire, which electrolytically disintegrates the solder junction, thereby detaching the coil from the delivery wire. The application of the current also creates a positive electrical charge on the coil, which attracts negatively-charged blood cells, platelets, and fibrinogen, thereby increasing the thrombogenicity of the coil. Several coils of different diameters and lengths can be packed into an aneurysm until the aneurysm is completely filled. The coils thus create and hold a thrombus within the aneurysm, inhibiting its displacement and its fragmentation.

The advantages of the GDC procedure are the ability to withdraw and relocate the coil if it migrates from its desired location, and the enhanced ability to promote the formation of a stable thrombus within the aneurysm.

While the microcoil-type vaso-occlusive devices of the prior art can be withdrawn and relocated, they may be prone to axial elongation ("stretching") and kinking during deployment, especially if a partial retrieval is needed to reposition the device. Such deformation of the vaso-occlusive device can result in the need to retrieve the device and to re-start the embolization procedure with a new device.

What is needed is a microcoil-type vaso-occlusive device with improved handling characteristics, which can resist stretching and kinking while maintaining a high level of flexibility during deployment and repositioning.

### SUMMARY OF THE INVENTION

The present invention provides an improved filamentous vaso-occlusive implant that resists stretching and kinking during deployment and repositioning within the vasculature.

Generally, the implant can be formed of at least two elongate coaxial members, including at least one inner member and a co-axially arranged outer member. Thus, the outer member has an interior surface defining a lumen in which the inner member is coaxially disposed. When placed in axial tension, the outer member radially contracts. The inner member provides radial resistance to counter the contraction of the outer member. Since the outer member is disposed coaxially around the inner member, once the radial contraction is impeded, elongation of the outer member is effectively inhibited. The amount of elongation will thus not exceed the elastic limit of the outer member under expected axial tension levels under normal operational conditions, so that permanent stretching or deformation will not take place under such conditions. Advantageously, this arrangement removes any requirement for having the inner member attached to the outer member. Beneficially, the shape of the inner member can be varied to provide increased resistance to contraction by the outer member, as detailed below.

In one aspect of the present invention, the two elongate members are formed of at least two helical coils of biocompatible metal wire. As best understood from the detailed description below, each coil can be formed of a multifilar configuration, or alternatively of a unifilar configuration. Advantageously, each individual filar can be made of the same or of different material, such as any biocompatible material known in the art of medical implants. Similarly, each filar can be formed with the same or with varying diameters of between about 0.0003 inches (0.008 mm) and about 0.012 inches (0.3 mm).

Each multifilar or unifilar coil can be wound in the same direction, or in opposite directions to provide less mechanical interference in motion between the two coils.

In another aspect of the present invention, the two elongate members are formed of at least two helical coils of biocompatible metal wire. Alternatively, one of the two elongate members may be made of any number of other suitable implant materials including polymers, collagen, proteins, drugs, and biological materials and combinations of these. Optionally, the inner member can be formed as a hollow tubular reservoir for transport and delivery of therapeutic compounds, bioactive agents, cellular material and the like.

In yet another aspect of the present invention, the inner and outer members may be made in various other flexible elongate configurations known in the art of vascular implants. These include, but are not limited to, cables, braids, and slotted or spiral cut tubes.

A small radial clearance can exist between the inner and outer members to allow a small amount of resistance-free stretching of the outer member before the resistance of the inner member is encountered when the outer member has radially contracted to contact the inner member. The size of this radial gap or clearance can be varied to provide different degrees of resistance-free stretching. Preferably, however, this gap or clearance will be no more than about 20% of the inside diameter of the outer member (that is, the diameter of the lumen), and, in some embodiments, there may be no clearance or gap at all.

The device advantageously includes a rounded or hemispherical distal tip and a proximal coupler, each being welded or soldered to its respective end of the device. The distal tip functions as an obturator to facilitate navigation through the tortuous bodily vasculature. The coupler provides an attachment mechanism to a delivery system and is detachable with the implant once ideal placement of the implant in a targeted vascular site is achieved.

In some embodiments of the invention, the inner member comprises at least two inner member segments separated by an empty area of the lumen, so that the device can exhibit varying degrees of flexibility along its length.

These and other features and advantages of the present invention will be more readily apparent from the detailed description of the embodiments set forth below taken in conjunction with the accompanying drawings.

### BRIEF DESCRIPTION OF THE DRAWINGS

FIG. 1A is an elevational views, partially in cross-section, of an implant in accordance with a first preferred embodiment of the present invention;
FIG. 1B is an elevational view, partially in cross-section, of a modification of the embodiment of FIG. 1A
FIG. 2 is a simplified perspective view of the implant of FIG. 1A showing the construction of the winding thereof;
FIG. 3 is a cross-sectional view taken along lines 3-3 of FIG. 2;
FIG. 4 is a cross-sectional view taken along line 4-4 of FIG. 3;
FIG. 5 is a cross-sectional view, similar to that of FIG 4, showing another modified form of the first preferred embodiment of the present invention ;
FIG. 6 is an axial cross-sectional view of a second preferred embodiment of the present invention;
FIG. 7 is a simplified perspective view of the implant of FIG. 1B showing the construction of the winding thereof;
FIG. 8 is a cross-sectional view, taken along line 8-8 of FIG. 7;
FIG. 9 is a cross-sectional view taken along line 9-9 of FIG. 8;
FIG. 10 is a simplified illustration of the implant in a secondary configuration in accordance with the preferred embodiments of the invention;
FIG. 11 is an elevational view, partially in cross section, of an implant having a single coil inner member in accordance with a preferred embodiment of the invention;
FIG. 12 is an elevational view, partially in cross section, of an implant having a double coil inner member in accordance with another preferred embodiment of the invention;
FIG. 13 is an elevational view, partially in cross section, of an implant having a cut or slotted tube inner member in accordance with still another preferred embodiment of the invention;
FIG. 14 is an elevational view, partially in cross section, of an implant having an inner member impregnated with a bioactive agent in accordance with another preferred embodiment of the invention;
FIG. 15 is an elevational view, partially in cross-section, of another preferred embodiment of the invention;
FIG. 16 is a cross-sectional view taken along line 19-19 of FIG. 15; and
FIG. 17 is an elevational view, partially in cross-section, of an embodiment of the invention that exhibits varying degrees of flexibility along its length.

### DETAILED DESCRIPTION OF THE INVENTION

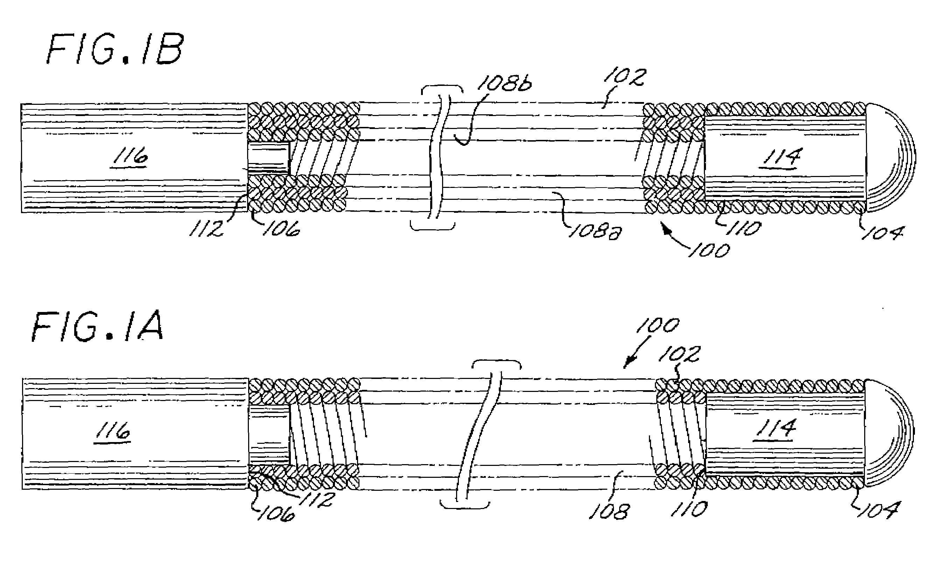

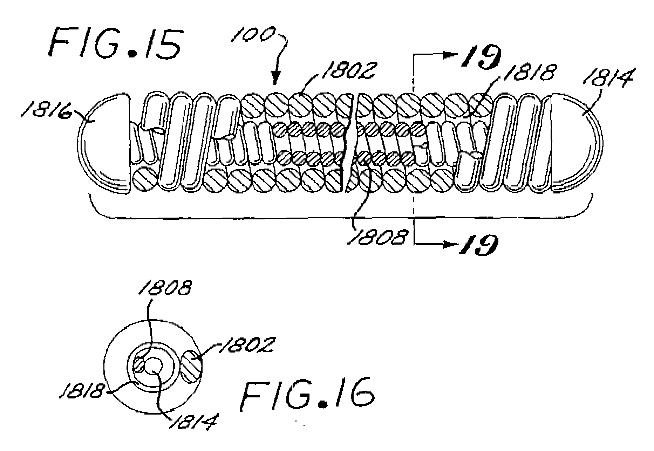

FIG. 1A shows an elevational view, partially in cross section, of an implant 100 in accordance with a preferred embodiment of the present invention. The implant 100 is an elongate, flexible, filamentous structure comprising an outer member 102 having a first or distal end 104 and a second or proximal end 106. The implant 100 also includes an inner member 108 having a first or distal end 110 and a second or proximal end 112. The inner member 108 is coaxially located within a lumen 113 defined by the interior surface of the outer member102. The implant 100 advantageously includes a rounded distal tip 114 and a proximal coupler 116, fixed to the distal end 104 and the proximal end 106 of the outer member 102, respectively, using conventional techniques, such as welding, gluing, brazing or soldering. The distal tip 114 functions as an obturator to facilitate navigation through the tortuous bodily vasculature during deployment of the implant. In some embodiments (see, e.g., Figures 15 and 16 and their description below), the distal end 110 of inner member 108 is not attached to the distal tip 114, thus leaving room for the inner member 108 to move freely relative to the outer member 102.

The proximal coupler 116 provides an attachment mechanism to a delivery mechanism (not shown) and is detachable with the implant 100 once ideal placement of the implant 100 in a target vascular site is achieved. In some embodiments, the proximal end 106 of the outer member 102 and the proximal end 112 of the inner member 108 are both fixed to the proximal coupler 116, while in other embodiments, the coupler 116 is fixed only to the proximal end of the outer member 102. Alternatively, the inner member 108 may be attached neither to distal tip 114 nor to the proximal coupler 116. This configuration allows the inner member108 to move relatively freely within the inner diameter of the outer member 102. Advantageously, this arrangement can improve the trackability of the implant 100. It should be understood, however, that the spaces between the unattached ends110 and 112 of the inner member 108, and distal tip 114 and the proximal coupler 116, respectively, are necessarily small to avoid leaving any significant portion of the outer member 102 unsupported.

In the preferred embodiments, the outer member 102 is formed as a helically wound microcoil. In the preferred embodiment, the inner member 108 is also formed of at least one helically wound inner microcoil. As shown in FIG. 1B, the inner member may also comprise first and second inner members 108a, 108b, either or both of which may be helically wound microcoils.

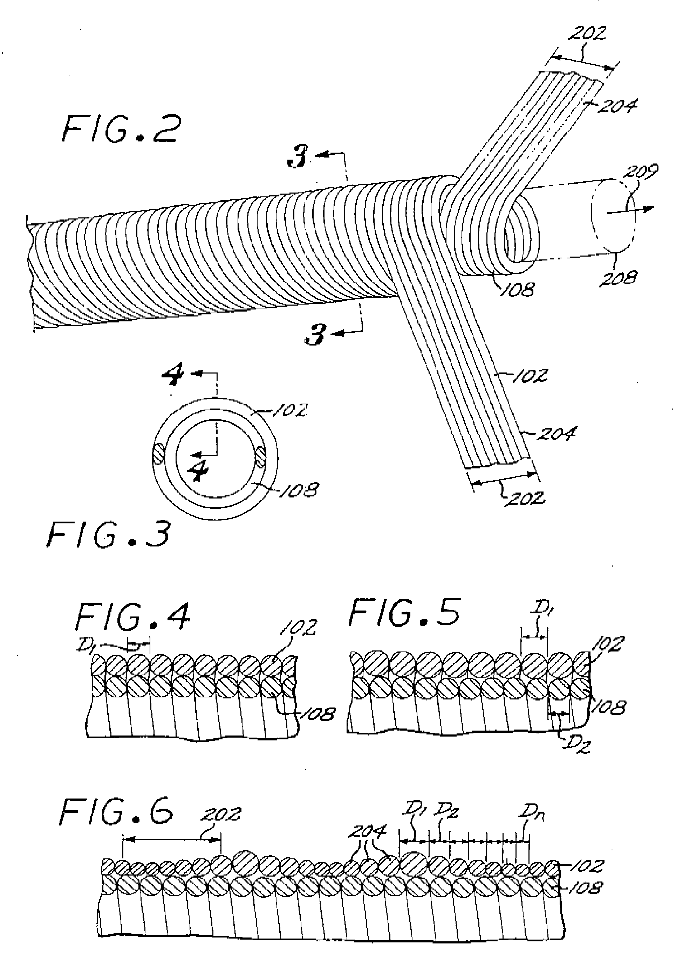

Referring now to FIG. 2, the outer member 102 and the inner member 108, if formed as microcoils, can each be formed of a multifilar winding, a unifilar winding, or a combination of both. A filar is a single filament of wire or other filamentous material. A unifilar winding comprises a single filament or filar, while the number of filars in a multifilar set can range from two to ten or more. Each filar set, whether unifilar or multifilar, can be wound in either Left-hand Wound (LHW) or Right-hand Wound (RHW) directions. When viewed from the end of the coil, the filars of a RHW coil wind in a clockwise (right) direction, as they rotate away from an observer's viewpoint. In a LHW coil, the filars wind in a counterclockwise (left) direction.

Several parameters of a microcoil can be customized for selected applications. Among these are the pitch of the filar configuration, measured from the beginning of one set of filars to the beginning of the next set of filars; the pitch of individual filars in each set; and the spacing between adjacent sets of filars. Multifilar and unifilar coils are commercially available from a number of sources that are known to those skilled in the art.

FIG. 2 shows an embodiment of the implant 100 formed of two microcoils, wherein the outer microcoil 102 and the inner microcoil 108 each comprise multifilar sets 202. In this exemplary embodiment, each set 202 includes eight filars 204; however any number of filars can be used.

Referring to FIGS. 2 and 3, to construct the implant 100, the inner microcoil 108 is first wound about a mandrel 208 or similar removable core. Alternatively, the inner microcoil 108 may be wound by a deflection winder (not shown) that does not require a core or mandrel. The inner microcoil 108 is typically oriented along a first angular bias relative to the central axis 209 of the mandrel 208 after it has been wound. Preferably, the inner and outer microcoils are wound independently, and the outer microcoil 102 is slid over the inner microcoil 108. Alternatively, the outer microcoil 102 may be wrapped around the inner microcoil 108. The outer microcoil 102 may be wound in either the same direction (bias) as is the inner microcoil 108 or in the alternate direction. The axial distance for one complete revolution of the filar set, that is, the "pitch" of the microcoil, is generally smaller for the outer microcoil 102 than for the inner microcoil 108, given similar filar diameters. Advantageously, the closer each filar set is to parallel with the axis 209 (i.e., the greater the pitch), the less slack is available for stretching.

In operation, when the outer microcoil 102 of the implant 100 is in tension, the outer microcoil 102 radially contracts. The inner microcoil 108 provides radial resistance to counter the contraction of the outer microcoil 102. The support provided by the inner microcoil 108 to the outer microcoil 102 resists the radial contraction of the outer microcoil 102; that is, the inner microcoil resists the tendency of the outer microcoil to decrease in diameter when under axial tension. Since the inner microcoil 108 does not easily yield to the contraction forces presented by the outer microcoil 102, the elongation of the implant 100 is limited so that it does not exceed the elastic limit of the outer microcoil 102, which thus does not permanently stretch or deform under a reasonable amount of load. Furthermore, the degree of axial elongation permitted by the dual microcoil structure does not significantly degrade the"pushability"of the implant; that is, its ability to be pushed through the vasculature (or other bodily lumen) without kinking or knotting is not significantly compromised.

FIG. 4 is a partial cross-sectional view of a section of FIG. 3, which illustrates that in some embodiments the filars of the outer microcoil 102 and the inner microcoil 108 can have a uniform diameter D₁. FIG. 5 is a partial cross section that illustrates that in some embodiments, the filars of the outer microcoil 102 can have a diameter D₁, while those of the inner microcoil can have a diameter D₂. In most embodiments, in which the outer microcoil 102 and inner microcoil 108 are formed of a metallic wire, the diameter of the filars 204 used in the production of the microcoils 102 and 108 is in the range of between about 0.0003 inches (0.008 mm) and about 0.012 inches (0.03 mm).

The outer microcoil 102 is wound with a primary diameter of between about 0.004 inches (0.1 mm) and about 0.04 inches (1 mm). The inner microcoil108 is sized to fit within the lumen 113 of the outer microcoil 102. In a preferred embodiment, a small radial gap or clearance 120 is allowed between the inner microcoil 108 and the outer microcoil 102. This gap or clearance would not typically exceed about 20% of the diameter of the lumen 113 (i.e., the inside diameter of the outer member or microcoil 102). Alternatively, there may be no gap or clearance between the inner or outer members, such as would be the case if the inner and outer members are respectively dimensioned so that there is an interference fit between them.

In one embodiment, as shown in FIG. 6, the outer microcoil102 can include sets 202 of filars 204. A filar set 202 may be one having a change in the outer diameter of the filars, generally a gradual transition from a large diameter to a small diameter. In this embodiment, the outer coil 102 includes filars 204 in a set 202 of varying diameters D₁, D₂... D_{N}. Friction is reduced between the outer microcoil 102 and the walls of a deployment catheter (not shown), since only those filars 204 with the largest diameter contact the walls of the catheter.

Each filar 204 can be made of a biocompatible metal. In addition, each filar 204 in a set 202 can be made of a different material, as well as having a different diameter. Each the microcoils 102 and 108 may be made of any of a wide variety of materials, such as a radio-opaque material including metals and polymers. Suitable metals and alloys include platinum, rhodium, palladium, rhenium, as well as tungsten, gold, silver, tantalum, and alloys of these metals. These metals have significant radiopacity and are also substantially biologically inert.

The filars 204 may also be of any of a wide variety of stainless steels and other materials which maintain their shape despite being subjected to high stress, such as nickel/titanium alloys, preferably the nickel/titanium alloy known as nitinol; platinum; tantalum; and various types of stainless steel that are known to be suitable for this type of application.

The filars 204 may be made of radiolucent fibers or polymers (or metallic threads coated with radiolucent or radiopaque fibers) such as Dacron (polyester), polyglycolic acid, polylactic acid,fluoropolymers (polytetrafluoro-ethylene), Nylon (polyamide), and silk. Should a polymer be used as the major component of the implant 100, it is desirably filled with some amount of a known radiopaque material, such as powdered tantalum, powdered tungsten, bismuth oxide, barium sulfate, and the like.

The axial length of the implant 100 can range between about 1 cm and about 100 cm, preferably between about 2 cm and about 60 cm. All of the dimensions here are provided only as guidelines and are not critical to the invention. However, only dimensions suitable for use in occluding sites within the human body are included in the scope of this invention.

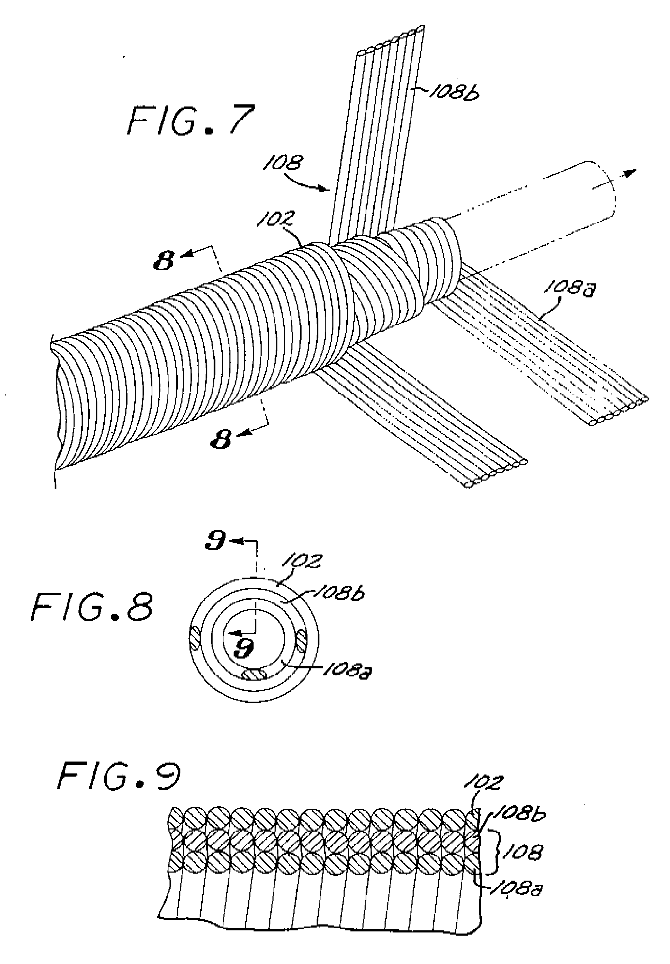

FIGS. 7, 8 and 9 illustrate yet another embodiment of the present invention. In this embodiment, the outer microcoil 102 and inner microcoil 108 perform the same function as in the earlier described embodiments. However, in this embodiment the inner microcoil 108 includes a first inner microcoil 108a and a second inner microcoil 108b. The inner microcoils 108a and 108b may formed around a mandrel 802, or they may be formed by a deflection winder (not shown) as discussed above. This "triple ply"-embodiment provides greater stretch resistance and more greater flexibility than the above-described "dual ply" embodiment of the same wall thickness.

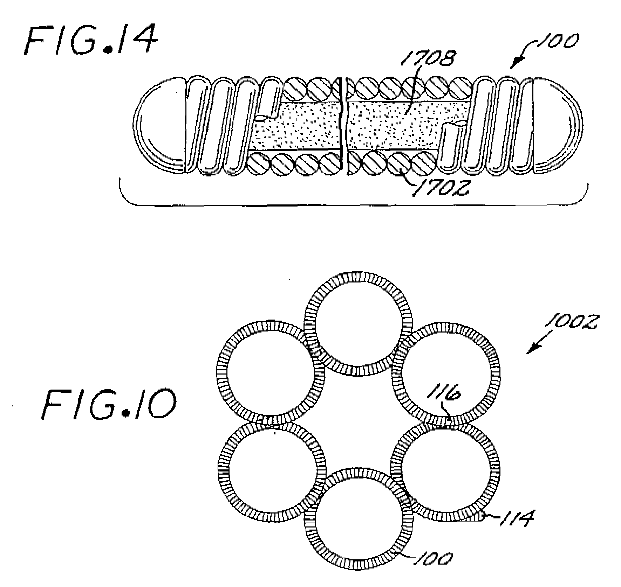

In a process well known in the art, as shown in FIG. 10, the implant 100 can be formed into a secondary configuration by heat treatment. The secondary configuration can be any shape deemed appropriate for a particular vascular treatment, such as a helical coil, sphere, ovoid, or other two dimensional and three dimensional shapes known in the art of vaso-occlusive devices. For example, as shown in FIG. 10, the implant 100 is annealed and then formed into the secondary configuration by winding or wrapping the implant 100 around a suitably shaped and sized mandrel (not shown) of refractory material. The resulting complex coil 1002 is then subjected to an annealing temperature for a specified period of time. In one embodiment, an implant, including the inner microcoil 102 and the outer microcoil 108 made of Pt/W, is heat set, as is well known in the art. For example, heat setting may be performed at a temperature of in the range of about 750 F (400 C) to about 1290 F (700 C), and the process may be performed for a duration in the range of about ten (10) to ninety (90) minutes, depending on the temperature, the coil diameter, and the filar diameter. After removing the implant 100 from the furnace, the implant is cooled and trimmed to length. The distal ball tip 114 and the proximal coupler 116 are. attached to the implant 100 at the appropriate ends. The implant 100 is then ultrasonically cleaned in a neutralizing solution. The complex coil configuration 1002 is thereby made permanent, and becomes the minimum energy state configuration of the implant 100.

The delivery of the implant 100 in the treatment of aneurysms or similar conditions can be accomplished using a variety of well known techniques. For example, the implant 100 can be delivered via a catheter in which the implant 100 is pushed through the catheter by a pusher. The distal tip or obturator 114 ensures a smooth traverse through the catheter lumen. The implant 100 passes through the lumen of the catheter in a linear shape and takes on a complex shape as originally formed after being deployed into the area of interest. Varieties of detachment mechanisms used to release the implant100 from a pusher can be coupled to the proximal coupler 116. These detachment mechanisms have been developed and are well known by those of ordinary skill in the art.

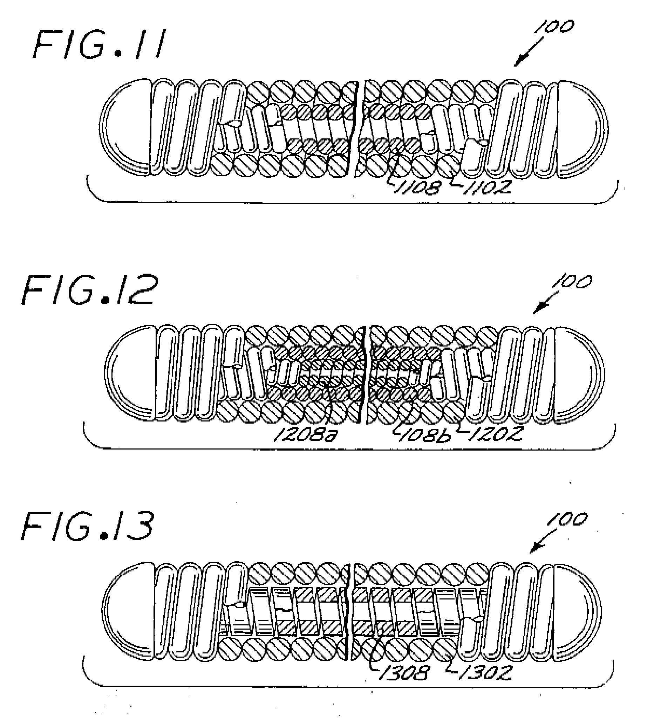

FIGS. 11-14 are partial cross sectional views of the implant 100 in accordance with various embodiments of the present invention. FIG. 11 illustrates an embodiment of the implant 100 including an outer microcoil 1102 wound about an inner microcoil 1108. In this embodiment, the outer microcoil 1102 is a multifilar configuration, which can have at least two and up to about ten filars, while the inner microcoil 1108 is a unifilar configuration. In this embodiment, the unifilar inner microcoil 1102 and each of the multifilars of the outer microcoil 1102 are Pt/W (preferably 92% Pt and 8% W) for enhanced radiopacity and minimal galvanic potential.

The inner and outer microcoils 1102 and 1108 can be LHW or RHW If the inner microcoil 1108 is RHW and the outer microcoil 1102 is LHW, then the two coils never interlock. The design of the implant 100 is more robust and tracks better through a tortuous vascular lumen when the inner microcoil 1108 and the outer microcoil 1102 are oriented opposite to one another.

FIG. 12 illustrates an embodiment of the implant 100 including an outer microcoil 1202 wound about an inner microcoil 1208, wherein the latter comprises a second inner microcoil 1208b wound about a first inner microcoil 1208a. It should be understood that the outer microcoil 1202 maybe made of other flexible, elongate configurations known in the art of vascular implants and instruments. These configurations may include ; but are not limited to cables, braids, and slotted or spiral cut tubes.

While the inner microcoils 1108 and 1208 are shown in FIGS. 11 and 12 to completely fill the space or lumen defined inside the outer microcoils 1102 and 1202, respectively, a small radial clearance may be allowed between the outer coils and the inner coils. In most embodiments, as mentioned above, the radial clearance would not exceed 20% of the inside diameter of the outer microcoil to ensure that the outer microcoils 1102 and 1202 are not allowed to stretch beyond a predetermined amount that is proportional to the width of the clearance.

FIG. 13 illustrates an embodiment of the implant 100 including an outer microcoil 1302 wound about an inner member 1308, wherein the latter comprises a spiral cut or slotted tube. The slotted tube 1308 provides rigidity while allowing for flexibility. This embodiment provides improved ability to transmit torque and greater axial strength.

FIG. 14 illustrates an embodiment of the implant 100 including an outer microcoil 1702 wound about an inner member 1708. In this embodiment, the inner member1708 can include a helical coil, cut or slotted tubes and any other appropriate carrier that can appropriately provide the function of the inner member 1708. The inner member 1708 may be coated, grafted, impregnated or otherwise made to carry a bioactive agent, which can be released while the implant 100 is at the target site within the vasculature. Appropriate bioactive agents can include, but are not limited to, thrombogenic agents, vasospasm inhibitors, calcium channel blockers, vasodilators, antihypertensive agents, antimicrobial agents, antibiotics, inhibitors of surface glycoprotein receptors, anti-inflammatory steroid or non- steroidal anti-inflammatory agents, immunosuppressive agents, growth hormone antagonists, growth factors, dopamine antagonists, radiotherapeutic agents, peptides, proteins, enzymes, extracellular matrix components, free radical scavengers, chelators, antioxidants, antipolymerases, antiviral agents, photodynamic therapy agents, cellular material, and gene therapy agents.

A composition can be prepared to include a solvent, a combination of complementary polymers dissolved in the solvent, and the bioactive agent or agents dispersed in the polymer/solvent mixture. The resultant composition can be applied to the inner member 1708 in any suitable fashion; for example, it can be applied directly to the surface of the inner member 1708 by dipping, spraying, or any conventional technique known to those of ordinary skill in the art. The method of applying the coating composition to the inner member 1708 will typically be governed by the geometry of the inner member and other process considerations. The coating is subsequently cured by evaporation of the solvent. An exemplary process for coating medical devices with a bioactive agent is disclosed in U.S. Patent No. 6,344,035 issued February 5, 2002, which is incorporated herein by reference for all purposes.

Figures 15 and 16 show another embodiment of the vaso-occlusive device 100 that includes an outer microcoil 1802 wound around an inner microcoil 1808. Both microcoils 1802, 1808 are attached at their respective proximal ends to a proximal coupling element 1814, while only the inner microcoil 1808 has a distal end that is attached to a distal obturator 1816. The inside diameter of the outer microcoil 1802 may advantageously be slightly larger than the outside diameter of the inner microcoil 1808, leaving a small radial gap or clearance 1818 between the two microcoils. As in the previously described embodiments, the radial gap 1818 preferably has a width of up to about 20% of the inside diameter of the outer coil 1802. This gap or clearance 1818 allows the outer microcoil 1802 to undergo a small amount of axial elongation or stretching before encountering the resistance offered by the inner microcoil 1808. The amount of elongation or stretching allowed is proportional to the width of the gap 1818. Accordingly, by varying the width of the gap 1818, the amount of stretching or elongation allowed can be adjusted. Of course, if little or no measurable stretching is desired, the respective diameters of the outer microcoil 1802 and the inner microcoil 1808 can be chosen so as to leave no gap between the two microcoils.

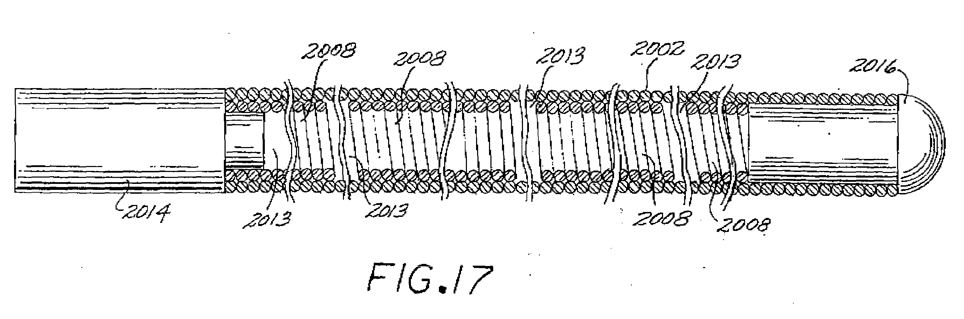

Figure 17 illustrates another embodiment of the vaso-occlusive device 100 that includes an outer element containing a coaxial inner element that occupies less than the entire length of the outer element. In the specific exemplary embodiment shown, the outer element is an outer microcoil 2002, while the inner element comprises at least two inner microcoil segments 2008 spaced apart longitudinally within the lumen 2013 of the outer microcoil 2002. The inner microcoil segments 2008 are separated by empty spaces within the lumen 2013. This arrangement provides the device 100 with varying degrees of flexibility along its length, with the portions of the device coinciding with the empty lumen spaces being more flexible than those portions in which the lumen 2013 contains an inner microcoil segment 2008. The inner microcoil segments 2008 may be of the same length or different lengths, as can be the empty lumen spaces. Alternatively, the inner element may be a single, unitary inner element (e.g., a microcoil) that occupies less than the full length of the outer element. As in the other embodiments, a coupling element 2014 is advantageously attached to the proximal end, while an obturator tip 2016 may be attached to the distal end.

The present invention exhibits several advantages over typical vaso-occlusive devices. For example, the implant 100 provides increased stretch resistance without sacrificing flexibility, and it does so with materials that are already known and approved for use in vascular implants. Furthermore, an implant constructed in accordance with the invention allows the implant to elongate slightly, to provide an indication that abnormal friction has been encountered, or that the device is knotted or trapped, while excessive elongation that would permanently deform or stretch the coil is resisted.

While embodiments of the invention have been described herein, it can be appreciated that variations and modifications will suggest themselves to those of ordinary skill in the art. For example, although the invention is described herein in the context of a vascular implant, it may be easily modified for use in occluding other bodily lumens, orifices, and passages. As a specific example, without limitation, the invention may be readily adapted for occluding a fallopian tube. Such modifications will readily suggest themselves to those skilled in the pertinent arts. For specific applications, the dimensions and materials may be varied from those disclosed herein if found to be advantageous. These and other variations and modifications are considered to be within the scope of the invention.

## Claims

1. An implant (100), comprising:
an elongate, flexible, hollow outer microcoil (102, 1102, 1202, 1302, 1702, 1802, 2002) having an interior surface defining a lumen (113, 2013), the outer microcoil (102, 1102, 1202, 1302, 1702, 1802, 2002) having a tendency to elongate axially and to contract radially when subject to axial tension; and
an inner member (108, 108a, 108b, 1108, 1208, 1208a, 1208b, 1308, 1708, 1808, 2008) disposed coaxially within the lumen (113, 2013) of the outer microcoil (102, 1102, 1202,1302, 1702, 1802, 2002) so as to resist the radial contraction of the outer microcoil (102, 1102, 1202, 1302, 1702, 1802, 2002) and thus to provide resistance to the axial elongation of the outer microcoil (102, 1102, 1202, 1302, 1702, 1802, 2002), **characterized in that** the inner member (108, 108a, 108b, 1108, 1208, 1208a, 1208b, 1308, 1708, 1808, 2008) comprises an inner microcoil (108, 108a, 108b, 1108, 1208, 1208a, 1208b, 1308, 1708, 1808, 2008).

2. The implant of Claim 1, wherein the inner microcoil (108, 108a, 108b, 1108, 1208, 1208a, 1208b, 1308,1708, 1808, 2008) comprises first (108a, 1208a) and second (108b, 1208b) inner microcoils disposed in a coaxial arrangement within the lumen (113, 2013) of the outer microcoil (102, 1102, 1202, 1302, 1702, 1802, 2002).

3. The implant of Claims 1 or 2, wherein the inner (108, 108a, 108b, 1108, 1208, 1208a, 1208b, 1308, 1708, 1808, 2008) and outer microcoils (102, 1102, 1202, 1302, 1702, 1802, 2002) are dimensioned so as to form a radial clearance (120, 1818) between them that is no more than about 20% of the diameter of the lumen (113, 2013).

4. The implant of Claims 1 or 2, wherein the inner (108, 108a, 108b, 1108, 1208, 1208a, 1208b, 1308, 1708, 1808, 2008) and outer microcoils (102, 1102, 1202, 1302, 1702, 1802, 2002) are dimensioned so as to leave substantially no radial clearance between them.

5. The implant of any of Claims 1-4, wherein the outer microcoil (102, 1102, 1202, 1302, 1702, 1802, 2002) comprises a multifilar winding

6. The implant of any of Claims 1-4, wherein the outer microcoil (102, 1102, 1202, 1302, 1702, 1802, 2002) comprises a unifilar winding.

7. The implant of any of Claims 1-6, wherein the inner microcoil (108, 108a, 108b, 1108, 1208, 1208a, 1208b, 1308, 1708, 1808, 2008) comprises a multifilar winding.

8. The implant of any of Claims 1-6, wherein the inner microcoil (108, 108a, 108b, 1108, 1208, 1208a, 1208b, 1308, 1708, 1808, 2008) comprises a unifilar winding.

9. The implant of any of Claims 1-8, wherein the inner microcoil (108, 108a, 108b, 1108, 1208, 1208a, 1208b, 1308, 1708, 1808, 2008) is provided with a bioactive agent.

10. The implant of any of Claims 1-9, wherein the inner microcoil (108, 108a, 108b, 1108, 1208, 1208a, 1208b, 1308, 1708, 1808, 2008) is dimensioned so as to have an interference fit within the lumen (113, 2013).

11. The implant of any of Claims 1-10, wherein the implant (100) exhibits varying degrees of flexibility along its length.

12. The implant of Claim 11, wherein the inner microcoil (108, 108a, 108b, 1108, 1208, 1208a, 1208b, 1308, 1708, 1808, 2008) comprises at least two inner segments separated by an empty area of the lumen (113, 2013).
